Europäisches Patentamt

European Patent Office          (11) Numéro de publication:  0 395 522

Office européen des brevets                                A1

(12)                DEMANDE DE BREVET EUROPEEN

(21) Numéro de dépôt: 90401141.8          (51) Int. Cl.5: A61B 6/00, G01T 1/29

(22) Date de dépôt: 26.04.90

(30) Priorité: 28.04.89 FR 8905663

(43) Date de publication de la demande:
31.10.90 Bulletin 90/44

(84) Etats contractants désignés:
DE GB NL

(71) Demandeur: GENERAL ELECTRIC CGR S.A.
100, rue Camille-Desmoulins
F-92130 Issy les Moulineaux(FR)

(72) Inventeur: Tirelli, Marco

Cabinet Ballot-Schmit, 7 rue le Sueur
F-75116 Paris(FR)
Inventeur: Romeas, René
Cabinet Ballot-Schmit, 7 rue le Sueur
F-75116 Paris(FR)
Inventeur: Gregoire, Yves
Cabinet Ballot-Schmit, 7 rue le Sueur
F-75116 Paris(FR)

(74) Mandataire: Ballot, Paul Denis Jacques et al
Cabinet Ballot-Schmit 7, rue le Sueur
F-75116 Paris(FR)

(54) Dispositif de contrôle de radiation à surface active modulable.

㊐ L'invention concerne un système de radiologie du type mammographe dans lequel il est prévu un contrôleur de radiation comportant un détecteur 22,23 de rayonnement X ayant traversé le sein à observer du type comportant un convertisseur de radiation 22 de rayonnement X en rayonnement photonique et un tube photomultiplicateur 23 des photons émis par le convertisseur. L'invention réside dans le fait que le convertisseur est masqué par une bande 29 opaque au rayonnement photonique sauf en des zones dont les dimensions variables sont prévues pour s'adapter aux différentes formes de seins. La bande 29 est entraînée par un moteur 34 et s'arrête sous la commande du praticien pour que la zone appropriée soit en correspondance avec le convertisseur 22.

FIG. 2

## DISPOSITIF DE CONTROLE DE RADIATION A SURFACE ACTIVE MODULABLE

L'invention concerne les systèmes de radiologie, notamment les mammographes et plus particulièrement dans de tels systèmes, un dispositif qui permet de contrôler la dose de radiation reçue par la personne en cours d'observation ainsi que la durée de la pose de manière à obtenir une image avec le contraste optimal.

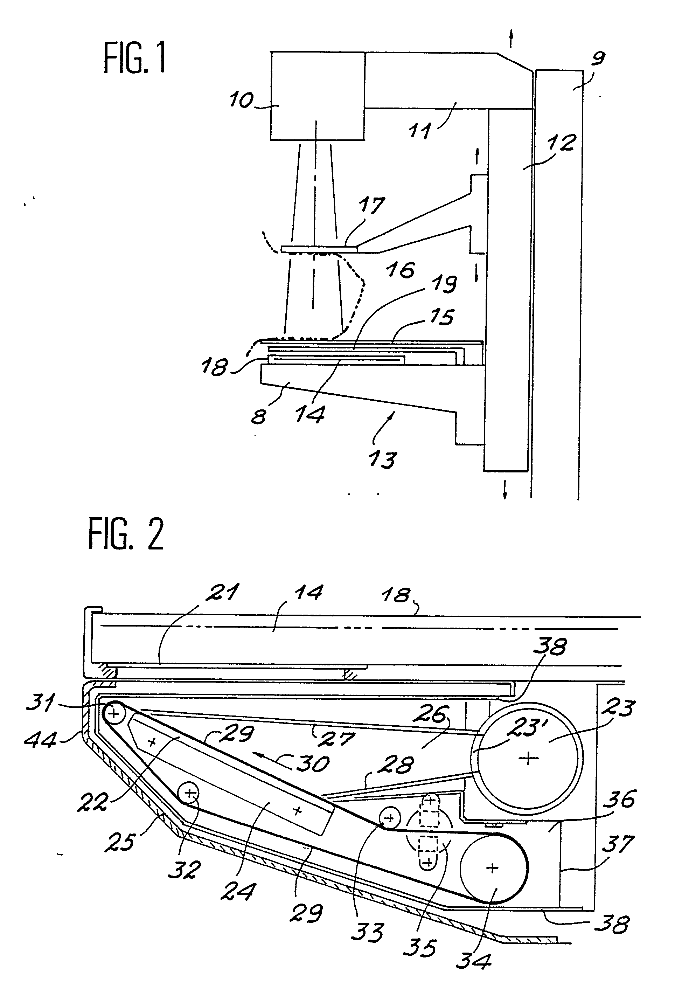

Les systèmes de radiologie du type mammographe comprennent, comme le montre la figure 1 une source 10 de rayonnement X portée par une potence 11 disposée au sommet d'une plaque verticale 12. Cette dernière comporte un ensemble 13 sur lequel repose le sein 16 à examiner par l'intermédiaire d'une tablette horizontale 15. Une pelote, transparente au rayonnement X et mobile verticalement sur la plaque 12, sert à comprimer le sein.

Pour s'adapter à la taille de la patiente, la plaque 12 est montée sur une colonne verticale 9 reposant sur le sol et se déplace verticalement sur ladite colonne grâce à un dispositif mécanique approprié.

L'ensemble 13 présente sur sa partie supérieure et sous la tablette 15, un tunnel dans lequel vient se loger une cassette 18 constituée d'une boîte noire renfermant un film sensible 14 au rayonnement X direct ou à un rayonnement photonique émis par un écran (non représenté) recevant le rayonnement X. C'est sur ce film 14 que se forme l'image latente du sein après une durée de pose appropriée; le développement du film donne un cliché radiographique.

Pour que le cliché présente un intérêt en vue d'un diagnostic, tous les points qui constituent l'image de l'objet examiné doivent présenter entre eux un contraste suffisant; en particulier, le noircissement du film doit être correct et "normalisé" pour un domaine très large d'opacité de l'objet. A cet effet, le noircissement peut être contrôlé par un dispositif de contrôle de radiation qui est disposé sous la cassette dans la partie inférieure 8 de l'ensemble 13. Ce dispositif de contrôle, également appelé exposeur, est constitué essentiellement d'un détecteur de rayonnement X qui délivre un signal électrique proportionnel au débit de la dose de rayonnement X qui passe à travers le film sensible. Ce signal électrique, qui traduit l'intensité du rayonnement X, est intégré pendant le temps de pose et le signal résultant de cette intégration est comparé à chaque instant à un signal de seuil pré-déterminé qui est fonction des caractéristiques du film sensible. Dès que le signal intégré atteint ce seuil, le signal indiquant l'égalité commande l'arrêt de la source , ce qui termine la pose.

Un des avantages de ce dispositif de contrôle de radiation est de permettre, pour une large plage de variation des débits de dose de rayonnement X qui conduisent à des différences de pose, d'une part, d'obtenir une exposition du film sensible correspondant à un contraste optimal et, d'autre part, de mieux contrôler la dose moyenne reçue par le patient, dose qui constitue un facteur important pour l'évaluation du risque carcinogène.

Dans un dispositif de contrôle de radiation, il est important que le détecteur ne reçoive que le rayonnement ayant traversé le sein car la réception d'un rayonnement X non atténué fausserait la mesure. Aussi, la surface réceptrice d'un tel détecteur est limitée par la taille du plus petit sein à examiner. Une telle limitation restreint considérablement les avantages que l'on peut tirer de ce dispositif et constitue un facteur d'erreur dans certaines circonstances car la zone de l'objet correspondant à la taille du détecteur peut être différente de celle que l'on examine. En effet, la position du détecteur est en général fixe alors que la zone à examiner peut avoir une position variable par rapport à celle du détecteur et il n'y a donc pas le recouvrement souhaité en vue d'une mesure optimale.

Cette limitation est encore plus marquée en mammographie car il y a une grande disparité entre les individus qui sont observés et, pour un même individu, une disparité selon le moment auquel est effectué l'examen par rapport au cycle hormonal. Dans la première catégorie, il y a les différences anatomiques comme la taille du sein ou la composition locale des tissus. Dans la deuxième catégorie, il y a la composition et la distribution des tissus en fonction du cycle hormonal, de l'age, du poids, du développement somatique. En outre, il y a la densité et la distribution des structures à visualiser, qu'elles soient pathologiques ou non, qu'elles soient massives ou qu'il s'agisse de microcalcifications dont les positions ne sont pas connues du praticien.

En résumé, avec un détecteur de position fixe et de petites dimensions, le signal de mesure n'est pas représentatif du sein dans son ensemble et peut conduire à des clichés sous-exposés dans le cas où le détecteur est sous une partie adipeuse du sein ou surexposés dans le cas où le détecteur est sous une partie fibreuse ou sous une opacité pathologique.

Par suite des insuffisances évoquées ci-dessus, les clichés obtenus seront difficilement exploitables par le praticien pour effectuer, avec un grand degré de certitude, un diagnostic ou un dépistage préventif. Il sera donc amené à refaire l'examen de sorte que les avantages de l'utilisation d'un détecteur, savoir la rapidité, le meilleur contraste, la réduction de la dose de rayonnement et du flou cinématique sont compromis. Ces inconvénients sont en partie atténués dans les systèmes où tout l'ensemble détecteur peut être déplacé dans son plan au-dessous du sein. Cependant, il existe une limite à la plus grande dimension possible du détecteur et, de ce fait, ce dernier est mal optimisé aux différentes dimensions de sein rencontrées. Le signal résultant de l'intégration est, dans ce cas, une approximation du signal optimum : c'est donc l'expérience qui doit guider le praticien dans le choix de la position du détecteur.

Par ailleurs, il n'est guère possible de prédire où seront les opacités sur le cliché, d'où la difficulté de choisir la position de la cellule au premier cliché.

Le but de la présente invention est donc de réaliser un dispositif de contrôle de radiation qui s'adapte aux différentes tailles de l'objet à radiographier, en particulier lorsqu'il s'agit d'un sein.

A cet effet, l'invention propose, d'une part, d'agrandir les dimensions du détecteur jusqu'à atteindre éventuellement celles du film sensible et, d'autre part, d'utiliser des masques de différentes dimensions qui sont placés entre la cassette et le détecteur.

L'invention se rapporte à un dispositif de contrôle de radiation dans un système de radiologie qui comprend au moins une source de rayonnement X et un détecteur du rayonnement X ayant traversé un objet à observer du type comportant un convertisseur de radiation de rayonnement X en rayonnement photonique et un tube photomultiplicateur des photons émis par le convertisseur, ledit détecteur fournissant un signal électrique qui sert à controler le temps de pose de l'objet, caractérisé en ce qu'il comprend, en outre, au moins un masque, opaque au rayonnement lumineux mais transparent au rayonnement X, qui est interposé entre le convertisseur de radiation et le photomultiplicateur, ledit masque présentant au moins une zone transparente au rayonnement lumineux, la forme et la surface de la zone transparente étant adaptées aux dimensions de l'objet à observer ou au type d'examen qui est effectué. Le masque est supporté par une bande qui est associée à un mécanisme de déplacement de ladite bande de manière à placer ladite zone transparente à une position optimale par rapport à l'objet.

En outre, cette bande comporte plusieurs zones transparentes au rayonnement lumineux qui sont disposées successivement dans le sens de déplacement de la bande et dont les dimensions et la forme sont différentes d'une zone à la suivante de manière à s'adapter aux dimensions et à la forme de l'objet ainsi qu'au type d'examen effectué.

D'autres caractéristiques et avantages de la présente invention apparaîtront à la lecture de la description suivante d'un exemple particulier de réalisation, ladite description étant faite en relation avec les dessins joints dans lesquels :

- la figure 1 est une vue en coupe verticale d'un mammographe dans un plan passant par la source du rayonnement X,

- la figure 2 est une vue en coupe verticale d'un exemple particulier de réalisation d'un dispositif automatique de changement de masque,

- la figure 3 est une vue déployée de la bande portant les différents masques,

L'invention sera décrite dans son application à un mammographe mais elle peut être mise en oeuvre dans d'autres systèmes de radiologie où il est fait usage d'un contrôleur de radiation du type comportant un convertisseur de radiation du rayonnement X en rayonnement photonique et un tube photomultiplicateur des photons émis par le convertisseur. Afin de mieux adapter le contrôle de radiation à la zone à examiner quelles que soient les dimensions du sein, l'invention propose de contrôler la surface de la zone active du convertisseur de radiation en interposant entre ce dernier et le tube photomultiplicateur un masque opaque au rayonnement lumineux qui présente des fenêtres transparentes dont les dimensions et la forme varient en fonction de celles du sein à examiner.

Pour effectuer une telle interposition de masques, l'invention propose un dispositif automatique de déplacement des masques.

Le dispositif automatique est disposé dans la partie inférieure 8 de l'ensemble 13 sous la cassette 18. Cette dernière comporte de manière connue, un film sensible 14 à l'intérieur de la boîte noire que constitue la cassette et une zone transparente 21 au rayonnement X qui est située dans la paroi inférieure de la cassette et à proximité du bord extérieur de la partie 8 le plus près du patient. Le convertisseur de radiation est constitué d'un écran 22 qui émet des photons vers un photomultiplicateur 23 lorsqu'il reçoit un rayonnement X traversant la zone transparente 21.

L'écran 22 est fixé sur un support 24 solidaire d'un châssis 25 constitué essentiellement par la partie inférieure 8 de l'ensemble 13. Les photons émis par l'écran 22 sont focalisés à l'entrée 23' du photomultiplicateur 23 par un conduit 26 comportant notamment les parois 27 et 28 opaques à la lumière mais transparentes aux rayonnement X.

Devant l'écran 22 est disposée une bande 29 qui est supportée et entraînée en mouvement suivant la flèche 30 par un moteur d'entraînement 34 et des galets de support 31, 32 et 33. Un mécanisme 35 permet de mettre en place la bande 29 et d'en régler la tension. La bande se déplace dans une boîte noire 36 comportant les parois 28, 37 et 38 de manière que la lumière extérieure ne vienne pas perturber la mesure du photomultiplicateur 23.

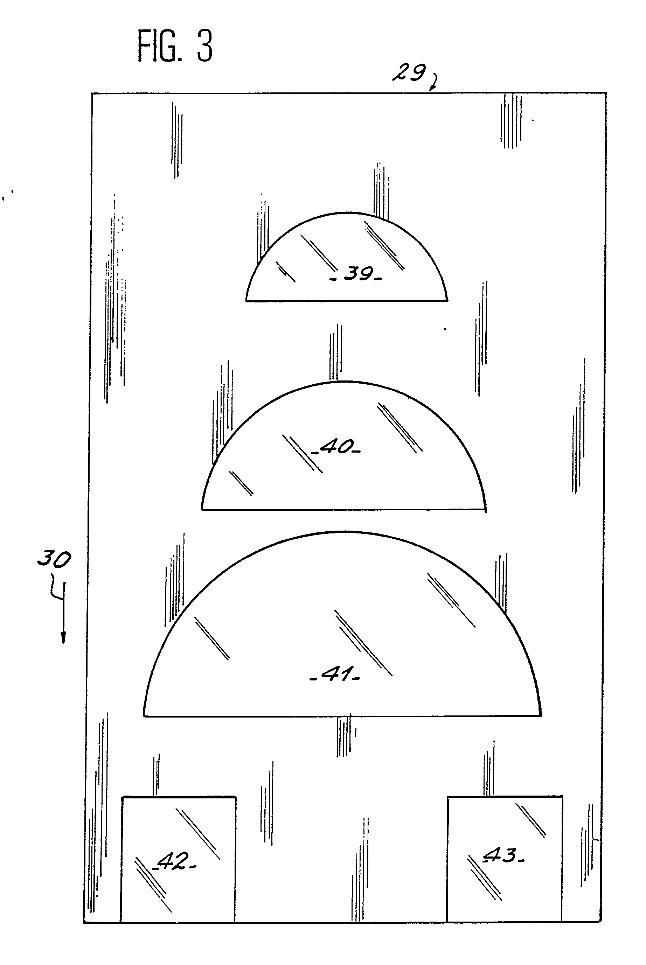

La bande 29 est refermée sur elle-même et est opaque au rayonnement lumineux sur toute sa surface à l'exception de zones 39,40,41,42 et 43 (figure 3). Les zones 39,40 et 41 ont une forme générale de demi-cercle dont le diamètre varie d'une zone à la suivante, la zone 39 de plus petit diamètre étant prévu par exemple pour l'examen d'un sein de petites dimensions, la zone 40 pour l'examen d'un sein de dimensions moyennes et la zone 41 pour l'examen d'un sein de grandes dimensions. Enfin, les zones 42 et 43, qui sont de forme rectangulaire et disposées symétriquement de part et d'autre de l'axe de symétrie longitudinale 44 de la bande, sont utilisées lors d'un examen stéréotaxique pour reproduire deux vues sur le même film.

Selon la longueur de la bande 29, il est possible de créer un nombre de fenêtres, telles que 39,40 et 41, supérieur à trois afin de mieux couvrir la disparité des dimensions des seins. En outre, ces zones peuvent avoir des formes autres que celle d'un demi-cercle. Bien entendu, les dimensions maximales de la fenêtre 41 ou du rectangle englobant les fenêtres 42 et 43 sont celles de l'écran 22 associé au photomultiplicateur 23.

Au cours d'un examen, le choix de la fenêtre qui est adaptée au sein observé est effectué par le praticien et le circuit de commande électrique du moteur d'entraînement 34 est prévu pour mettre en place la fenêtre choisie devant l'écran 22, la base du demi-cercle étant située vers le bord extérieur 44 de la partie 8 de l'ensemble 13, le plus près possible du patient.

L'invention qui vient d'être décrite permet donc au praticien d'adapter le temps de pose et donc la dose de radiation émise en fonction, notamment, des dimensions du sein à examiner.

## Revendications

1. Dispositif de contrôle de radiation dans un système de radiologie qui comprend au moins une source (10) de rayonnement X et un détecteur (22,23) de rayonnement X ayant traversé l'objet à observer, du type comportant un convertisseur de radiation (22) de rayonnement X en rayonnement photonique et un tube photomultiplicateur (23) des photons émis par le convertisseur (22), ledit détecteur fournissant un signal électrique qui sert à contrôler le temps de pose de l'objet, caractérisé en ce qu'il comprend, en outre, au moins un masque, opaque au rayonnement lumineux mais transparent au rayonnement X, qui est interposé entre le convertisseur de radiation (22) et le photomultiplicateur (23), ledit masque présentant au moins une zone transparente au rayonnement lumineux, la forme et la surface de la zone (39,40,41,42,43) transparente étant adaptées aux dimensions de l'objet à observer ou au type d'examen qui est effectué.

2. Dispositif de contrôle selon la revendication 1, caractérisé en ce que le masque est supporté par une bande (29) qui est associée à un mécanisme de déplacement (31 à 34) de ladite bande de manière à placer ladite zone transparente à une position optimale par rapport à l'objet.

3. Dispositif de contrôle selon la revendication 2, caractérisé en ce que la bande comporte plusieurs zones transparentes (39 à 43) au rayonnement lumineux, qui sont disposées successivement dans le sens de déplacement de la bande et dont les dimensions et la forme sont différentes d'une zone à la suivante de manière à s'adapter aux dimensions et à la forme de l'objet.

FIG. 1

FIG. 2

# FIG. 3

Office européen
des brevets

**RAPPORT DE RECHERCHE EUROPEENNE**

Numero de la demande

EP 90 40 1141

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 158 838 (FUJI PHOTO FILM CO., LTD)<br>* Page 19, ligne 20 - page 20, ligne 9; page 24, ligne 21 - page 27, ligne 21; figures 1-9 *<br>--- | 1,3 | A 61 B 6/00<br>G 01 T 1/29 |
| A | US-A-3 824 397 (BAUER et al.)<br>* Colonne 1, ligne 50 - colonne 2, ligne 39; fig. *<br>----- | 1 | |

DOMAINES TECHNIQUES RECHERCHES (Int. Cl.5)

G 01 T
A 61 B

Le présent rapport a été établi pour toutes les revendications

| Lieu de la recherche | Date d'achèvement de la recherche | Examinateur |
|---|---|---|
| LA HAYE | 07-08-1990 | DATTA S. |